(19) Europäisches Patentamt
European Patent Office
Office européen des brevets

(11) Veröffentlichungsnummer: **0 553 630 A1**

(12) # EUROPÄISCHE PATENTANMELDUNG

(21) Anmeldenummer: **93100337.0**

(22) Anmeldetag: **12.01.93**

(51) Int. Cl.[5]: **C07D 213/64**, C07D 405/12, C07F 7/10, A01N 43/40, A01N 43/08, //(C07D405/12, 307:00,213:00)

(30) Priorität: **25.01.92 DE 4202053**

(43) Veröffentlichungstag der Anmeldung:
**04.08.93 Patentblatt 93/31**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Andree, Roland, Dr.**
**Dechant-Miebach-Weg 37**
**W-4018 Langenfeld(DE)**
Erfinder: **Haug, Michael, Dr.**
**Fahner Weg 5**
**W-5060 Bergisch Gladbach 2(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Pyridyloxy-naphthaline als Herbizide.**

(57) Beschrieben werden neue Pyridyloxy-naphrthaline der Formel (I)

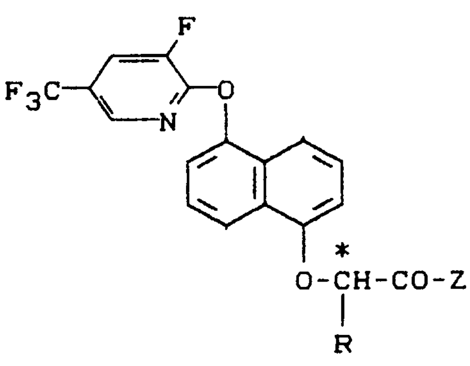

(I)

in welcher
R und Z die in der Beschreibung angegebene Bedeutung haben sowie mehrere Verfahren zu deren Herstellung.
Die neuen Pyridyloxy-naphthaline werden als Herbizide verwendet.

EP 0 553 630 A1

Die Erfindung betrifft neue Pyridyloxy-naphthaline, Verfahren zu ihrer Herstellung und ihre Verwendung als Herbizide.

Es ist bereits bekannt, daß bestimmte Pyridyloxy-naphthaline, wie z.B. α-(5-(3,5-Dichlor-pyridin-2-yl-oxy)-naphthalin-1-yl-oxy)-propionsäure-ethylester und α(5-(3-Chlor-5-trifluormethyl-pyridin-2-yl-oxy)-naphthalin-1-yl-oxy)-propionsäure-ethylester, herbizide Eigenschaften aufweisen (vgl. JP-A 54032477 - zitiert in Chem. Abstracts 91:91510j). Die herbizide Wirksamkeit dieser bekannten Verbindungen ist jedoch nicht immer ganz zufriedenstellend.

Es wurden nun neue Pyridyloxy-naphthaline der allgemeinen Formel (I) gefunden,

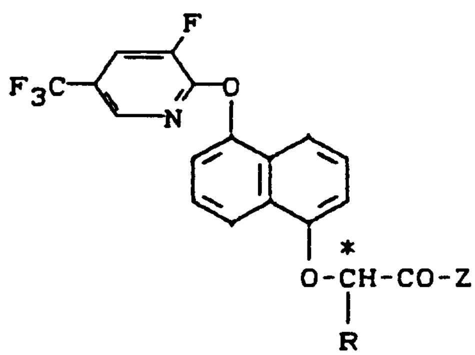

(I)

in welcher

R für Wasserstoff oder $C_1$-$C_4$-Alkyl steht und

Z für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, N-($C_1$-$C_6$-Alkoxy)-N-($C_1$-$C_4$-alkyl)-amino, Hydrazino, $C_1$-$C_4$-Alkylsulfonylhydrazino, Phenylsulfonylhydrazino, Tolysulfonylhydrazino, $C_1$-$C_4$-Alkylthio, Phenylthio, Benzylthio, $C_1$-$C_4$-Alkoxycarbonyl-$C_1$-$C_2$-alkylthio oder für die Gruppierung -O-$R^1$ steht, worin

$R^1$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl, $C_1$-$C_4$-Alkyl-sulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Trimethylsilylethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino, $C_2$-$C_4$-Alkylidenaminooxy-$C_1$-$C_4$-alkyl, oder für ein Ammonium-, ein $C_1$-$C_4$-Alkylammonium-, ein Natrium-, Kalium- oder Calcium-äquivalent steht, oder für die Gruppierung

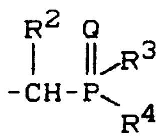

steht, worin

$R^2$ für Wasserstoff, $C_1$-$C_4$-Alkyl, Phenyl, Furyl, Thienyl oder Pyridyl steht,

$R^3$ für $C_1$-$C_4$-Alkyl oder $C_1$-$C_4$-Alkoxy steht,

$R^4$ für $C_1$-$C_4$-Alkoxy steht und

Q für Sauerstoff oder Schwefel steht,

oder

$R^1$ für die Gruppierung $(-CH_2-)_n-R^5$ steht, worin

n für die Zahlen 0, 1 oder 2 steht und

$R^5$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Isoxazolidinyl, Pyridinyl oder Pyrimidinyl steht.

Die Verbindungen der Formel (I) enthalten gegebenenfalls ein asymmetrisch substituiertes Kohlenstoffatom (wenn R≠H) und können dann in verschiedenen enantiomeren Formen vorliegen. Die Erfindung betrifft sowohl die möglichen einzelnen Isomeren als auch Gemische dieser Isomeren.

Weiter wurde gefunden, daß man die neuen Pyridyloxynaphthaline der Formel (I) erhält, wenn man

(a) 5-(3-Fluor-5-trifluormethyl-pyridin-2-yl-oxy)-1-naphthol der Formel (II)

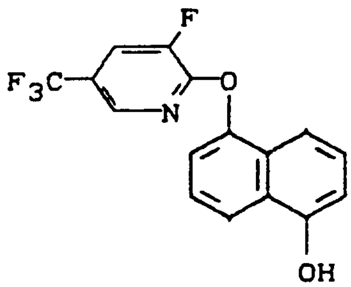

(II)

mit Carbonsäurederivaten der allgemeinen Formel (III)

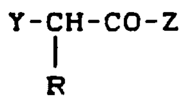

(III)

in welcher

R und Z die oben angegebene Bedeutung haben und

Y für eine nucleophile Abgangsgruppe steht,

gegebenenfalls in Gegenwart eines Säureakzeptors und gegebenenfalls in Gegenwart eines Verdünnungsmittels umsetzt, oder wenn man

(b) Halogen-pyridin-Derivate der allgemeinen Formel (IV)

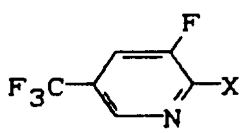

(IV)

in welcher

X für Halogen steht,

mit Hydroxynaphthyloxycarbonsäure-Derivaten der allgemeinen Formel (V)

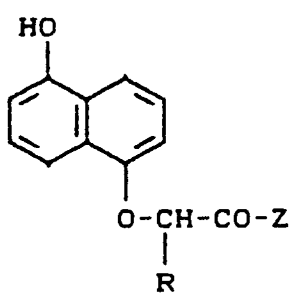

(V)

in welcher

R und Z die oben angegebene Bedeutung haben,

gegebenenfalls in Gegenwart eines Säureakzeptors und gegebenenfalls in Gegenwart eines Verdünnungsmittels umsetzt, oder wenn man

(c) für den Fall, daß Z für Hydroxy steht, die Verbindungen der Formel (I), in welcher Z für Methoxy oder Ethoxy steht, mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder wenn man

(d) für den Fall, daß Z für Chlor steht, die Verbindungen der Formel (I), in welcher Z für Hydroxy steht, mit einem Chlorierungsmittel gegebenenfalls in Gegenwart eines Katalysators und gegebenenfalls in Gegenwart eines Verdünnungsmittels umsetzt, oder wenn man

(e) für den Fall, daß Z mit Ausnahme von Chlor die oben angegebene Bedeutung hat, die Verbindungen der Formel (I), in welcher Z für Chlor steht, mit Verbindungen der allgemeinen Formel (VI)

H-Z (VI)

in welcher

Z mit Ausnahme von Chlor die oben angegebene Bedeutung hat,

gegebenenfalls in Gegenwart eines Säureakzeptors und gegebenenfalls in Gegenwart eines Verdünnungsmittels umsetzt, oder wenn man

(f) für den Fall, daß Z für die Gruppierung $-O-R^1$ steht, worin $R^1$ mit Ausnahme von Ammonium, Alkylammonium, Alkalimetall und Erdalkalimetall die oben angegebene Bedeutung hat, Verbindungen der Formel (I), in welcher Z für Hydroxy, Methoxy oder Ethoxy steht,

mit Hydroxyverbindungen der allgemeinen Formel (VII)

$HO-R^1$ (VII)

in welcher

$R^1$ mit Ausnahme von Ammonium, Alkylammonium, Alkalimetall und Erdalkalimetall die oben angegebene Bedeutung hat,

gegebenenfalls in Gegenwart eines Katalysators und gegebenenfalls in Gegenwart eines Verdünnungsmittels umsetzt.

Die neuen Pyridyloxy-naphthaline der allgemeinen Formel (I) zeichnen sich durch starke herbizide Wirksamkeit aus.

Überraschenderweise zeigen die erfindungsgemäßen Verbindungen der Formel (I) bei guter Kulturpflanzenverträglichkeit wesentlich stärkere Wirkung gegen bestimmte Problemunkräuter als die Verbindungen α-(5-(3,5-Dichlorpyridin-2-yl-oxy)-naphthalin-1-yl-oxy)-propionsäureethylester und α-(5-(3-Chlor-5-trifluormethyl-pyridin-2-yl-oxy)-naphthalin-1-yl-oxy)-propionsäure-ethylester, welche strukturell ähnliche vorbekannte Wirkstoffe gleicher Wirkungsrichtung sind.

Die Erfindung betrifft vorzugsweise Verbindungen der Formel (I), in welcher

R für Wasserstoff, Methyl oder Ethyl steht und

Z für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, N-($C_1$-$C_4$-Alkoxy)-N-($C_1$-$C_3$-alkyl)-amino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^1$ steht, worin

$R^1$ für $C_1$-$C_4$-Alkyl, $C_1$-$C_2$-Alkoxy-$C_1$-$C_2$-alkyl, $C_1$-$C_2$-Alkoxy-$C_1$-$C_2$-alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl, Trimethylsilylethyl, $C_2$-$C_4$-Alkylidenaminooxy-$C_2$-$C_3$-alkyl oder für ein Ammonium-, $C_1$-$C_3$-Alkylammonium-, Natrium- oder Kalium-äquivalent steht,

oder für

die Gruppierung

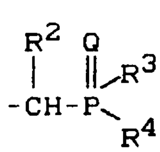

steht,
worin

$R^2$ für Wasserstoff, Methyl, Phenyl, Furyl, Thienyl oder Pyridyl steht,
$R^3$ für Methoxy oder Ethoxy steht,
$R^4$ für Methoxy oder Ethoxy steht und
Q für Sauerstoff oder Schwefel steht,
oder
$R^1$ für die Gruppierung $-(CH_2)_n-R^5$ steht, worin
n für die Zahlen 0, 1 oder 2 steht und
$R^5$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl, Isoxazolidinyl und Dioxolanyl steht.

Verwendet man für das erfindungsgemäße Verfahren (a) beispielsweise 5-3(Fluor-5-trifluormethyl-pyridin-2-yl-oxy)-1-naphthol und α-Brom-propionsäure-ethylester als Ausgangsstoffe, so kann der Reaktionsablauf durch das folgende Formelschema skizziert werden:

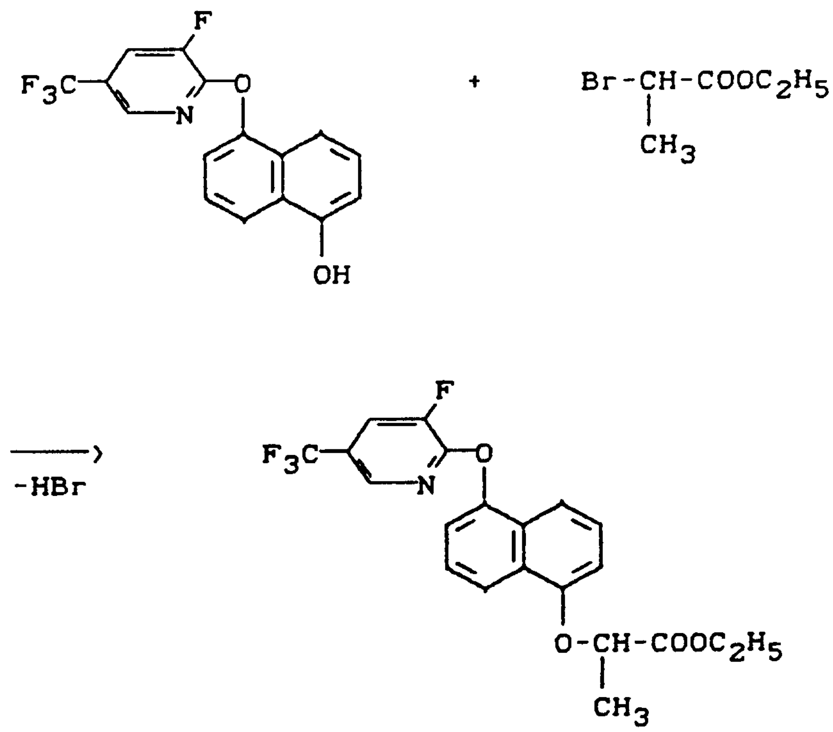


Verwendet man für das erfindungsgemäße Verfahren (b) beispielsweise 2,3-Difluor-5-trifluormethyl-pyridin und α-(5-Hydroxy-naphthalin-1-yl-oxy)-buttersäure-methylester als Ausgangsstoffe, so kann der Reaktionsablauf durch das folgende Formelschema skizziert werden;

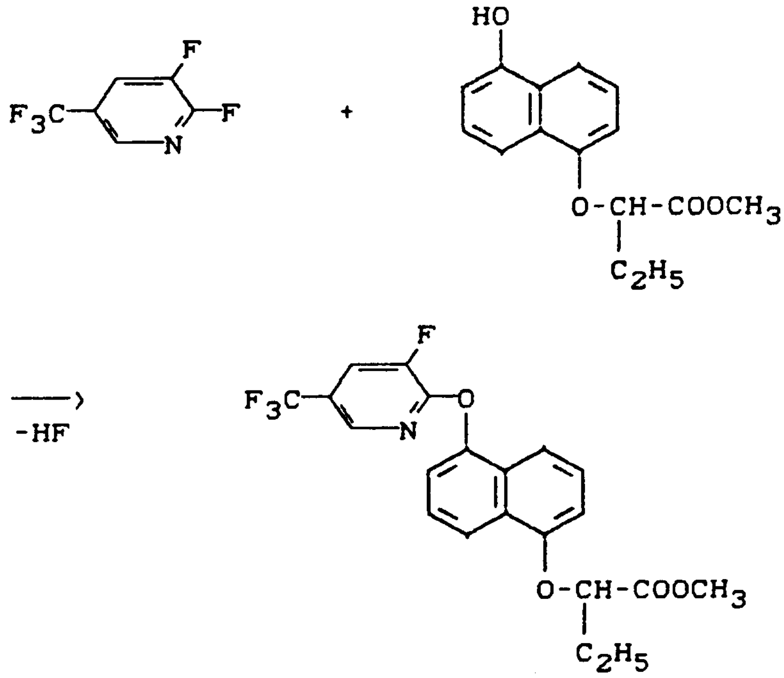


Verwendet man für das erfindungsgemäße Verfahren (c) beispielsweise α-(5-(3-Fluor-5-trifluormethyl-pyridin-2-yl-oxy)-naphthalin-1-yl-oxy)-essigsäure-methylester und wäßrige Natronlauge als Ausgangsstoffe, so kann der Reaktionsablauf durch das folgende Formelschema skizziert werden:

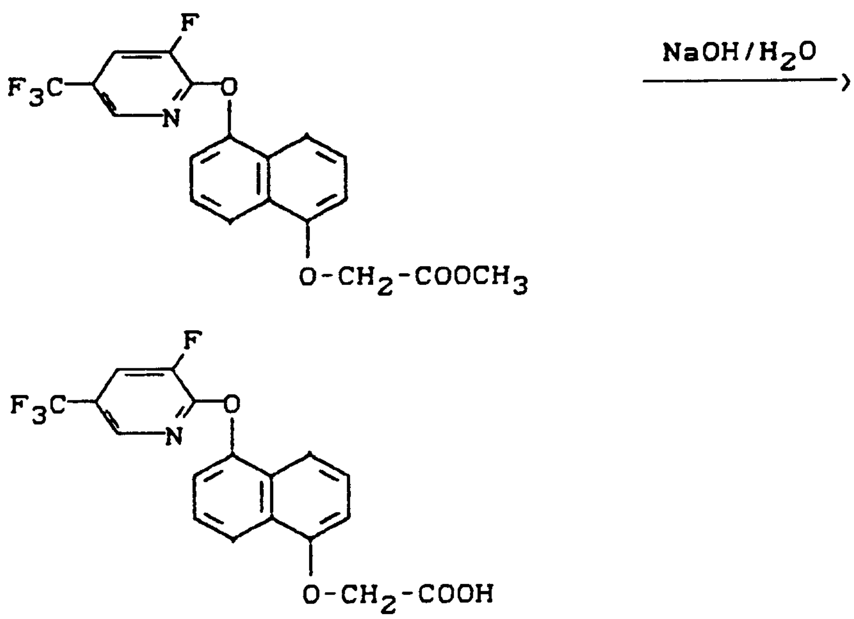


Verwendet man für das erfindungsgemäße Verfahren (d) beispielsweise α-(5-(3-Fluor-5-trifluormethylpyridin-2-yl-oxy)-naphthalin-1-yl-oxy)essigsäure und Thionylchlorid als Ausgangsstoffe, so kann der Reaktionsablauf durch das folgende Formelschema skizziert werden:

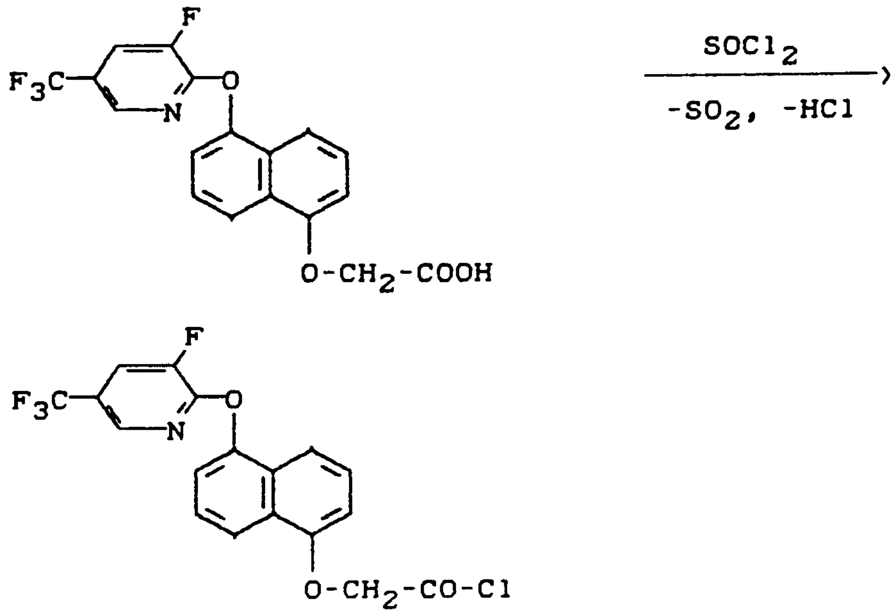


Verwendet man für das erfindungsgemäße Verfahren (e) beispielsweise α-(5-(3-Fluor-5-trifluormethyl-pyridin-2-yl-oxy)-naphthalin-1-yl-oxy)-propionsäurechlorid und Butanol als Ausgangsstoffe, so kann der Reaktionsablauf durch das folgende Formelschema skizziert werden:

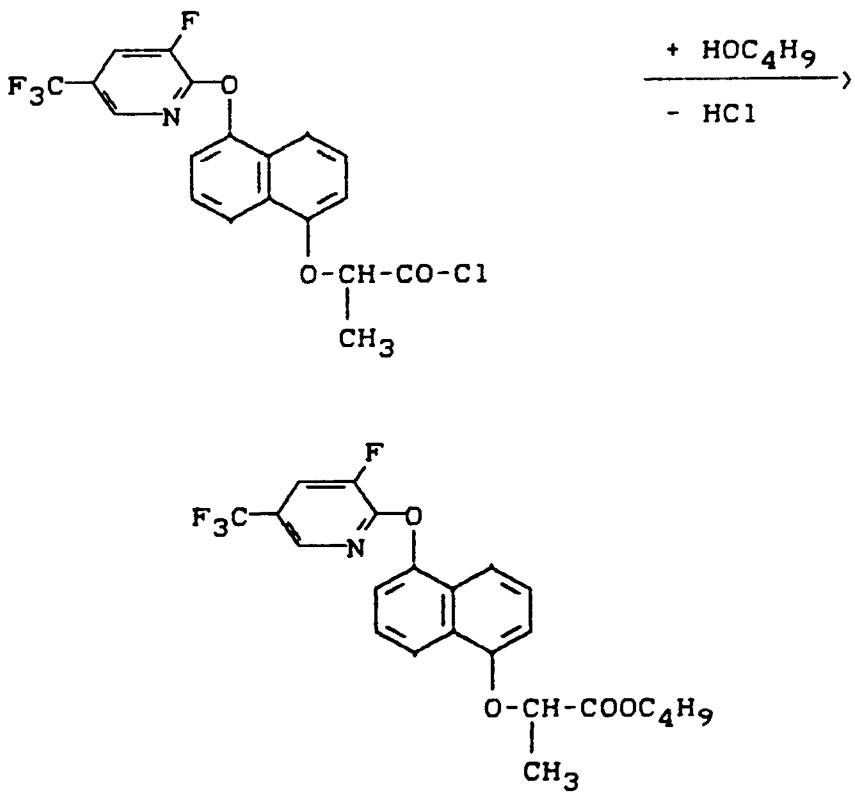


Verwendet man für das erfindungsgemäße Verfahren (f) beispielsweise α-(5-(3-Fluor-5-trifluormethyl-pyridin-2-yl-oxy)-naphthalin-1-yl-oxy)propionsäure und Propanol als Ausgangsstoffe, so kann der Reaktionsablauf durch das folgende Formelschema skizziert werden:

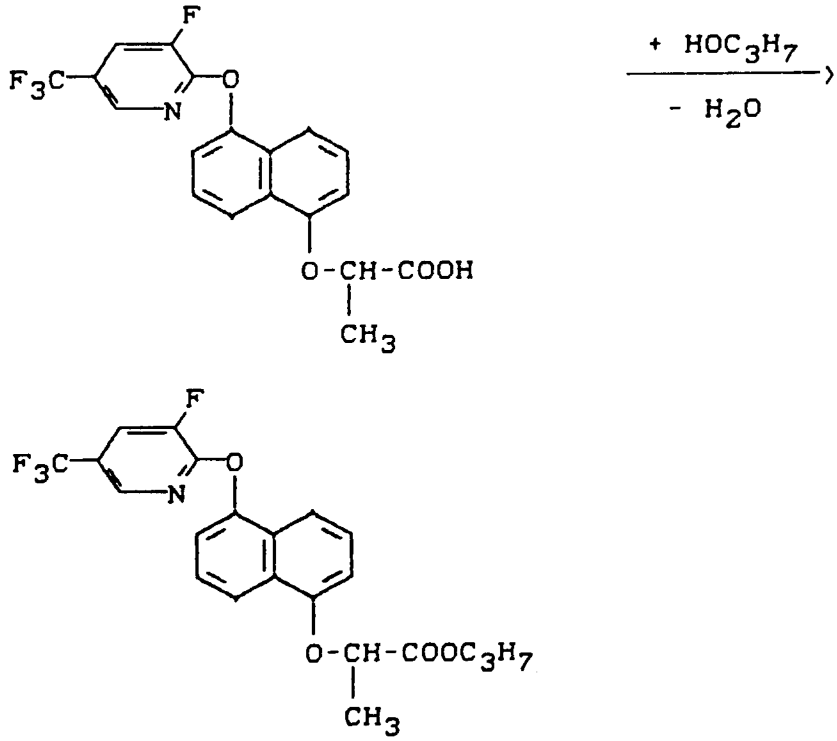


Das beim erfindungsgemäßen Verfahren (a) als Ausgangsverbindung zu verwendende 5-(3-Fluor-5-trifluormethylpyridin-2-yl-oxy)-1-naphthol der Formel (II) ist noch nicht aus der Literatur bekannt und ist als neue Verbindung Gegenstand der vorliegenden Patentanmeldung.

Man erhält die neue Verbindung der Formel (II), wenn man Halogen-pyridin-Derivate der allgemeinen Formel (IV)

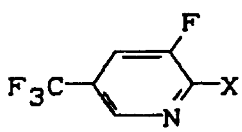

(IV)

in welcher

X für Halogen, vorzugsweise für Fluor oder Chlor steht,

mit 1,5-Dihydroxynaphthalin in Gegenwart eines Säureakzeptors, wie z.B. Natriumhydroxid oder Kaliumhydroxid, und in Gegenwart eines Verdünnungsmittels, wie z.B. Dimethylformamid, Dimethylacetamid, Dimethylsulfoxid, Tetramethylensulfon oder N-Methyl-pyrrolidon, bei Temperaturen zwischen 20 °C und 150 °C umsetzt und nach üblichen Methoden aufarbeitet. Die Halogen-pyridin-Derivate der Formel (IV) sind bereits bekannt (vgl. EP-A 146924, EP-A 178260, US-P 4563530, US-P 4680406).

Die beim erfindungsgemäßen Verfahren (a) weiter als Ausgangsstoffe zu verwendenden Carbonsäure-Derivate sind durch die Formel (III) allgemein definiert. In Formel (III) haben R und Z vorzugsweise diejenige Bedeutung, die bereits oben im Zusammenhang mit der Beschreibung der erfindungsgemäßen Verbindungen der Formel (I) vorzugsweise für R und Z angegeben wurde und Y steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Als Beispiele für die Verbindungen der Formel (III) seien genannt;

α-Chlor-, α-Brom- und α-Iod-essigsäure-, -propionsäure- und und -buttersäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenylsulfonyloxy)-essigsäure-, -propionsäure- und -buttersäure-methylester, -ethylester, -propylester, -butylester, -isopropylester, -isobutylester und -sec-butylester.

Unter den genannten Verbindungen der Formel (III) sind jeweils gegebenenfalls die R-Isomeren, die S-Isomeren und die racemischen Gemische dieser Isomeren zu verstehen.

Die Ausgangsstoffe der Formel (III) sind bekannt und/ oder können nach an sich bekannten Verfahren hergestellt werden (vgl. DE-OS 27 58 002, DE-OS 28 54 542).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden.

Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) als Ausgangsstoffe zu verwendenden Halogen-pyridin-Derivate der Formel (IV) wurden bereits oben beschriebene

Die beim erfindungsgemäßen Verfahren (b) weiter als Ausgangsstoffe zu verwendenden α-(5-Hydroxy-naphthalin-1-yl-oxy)-carbonsäure-Derivate sind durch die Formel (V) allgemein definiert. In Formel (V) haben R und Z vorzugsweise diejenige Bedeutung, die bereits oben im Zusammenhang mit der Beschreibung der erfindungsgemäßen Verbindungen der Formel (I) vorzugsweise für R und Z angegeben wurde,

Als Beispiele für die Ausgangsstoffe der Formel (V) seien genannt:

α-(5-Hydroxy-naphthalin-1-yl-oxy)essigsäure-, -propionsäure- und -buttersäure-methylester, -ethylester, -propylester, -isopropylester, -butylester, -isobutylester und -sec-butylester.

Die Ausgangsstoffe der Formel (V) sind bekannt und/oder können nach an sich bekannten Verfahren hergestellt werden (vgl. JP 79/32477, zit. in Chem. Abstracts 91 (1979), 91510j).

Verfahren (b) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden. Aprotisch polare organische Lösungsmittel, wie z. B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Verfahren (b) wird vorzugsweise in Gegenwart eines Säureakzeptors duchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Als Ausgangsstoffe für das erfindungsgemäße Verfahren (c) seien genannt:
α-(5-(3-Fluor-5-trifluormethyl-pyridin-2-yl-oxy)-naphthalin-1-yl-oxy)-essigsäure-, -propionsäure- und -buttersäure-methylester und -ethylester.

Die Ausgangsstoffe der Formel (I) für Verfahren (c) sind erfindungsgemäße neue Verbindungen; sie können nach den erfindungsgemäßen Verfahren (a) oder (b) hergestellt werden.

Verfahren (c) wird unter Verwendung von Alkalihydroxiden durchgeführt. Als Beispiel hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol eingesetzt.

Zum Ansäuern werden bei Verfahren (c) die üblichen Mineralsäuren, wie z. B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Ausgangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird gegebenenfalls bei erhöhter Temperatur, bis zum Reaktionsende gerührt. Das - gegebenenfalls nach Einengen, Abkühlen und Ansäuern - kristallin anfallende Reaktionsprodukt kann durch Absaugen isoliert werden.

Als Ausgangsstoffe für das erfindungsgemäße Verfahren (d) seien genannt:
α-(5-(3-Fluor-5-trifluormethyl-pyridin-2-yl-oxy)-naphthalin-1-yl-oxy)-essigsäure, -propionsäure und -buttersäure.

Diese Ausgangsverbindungen für Verfahren (d) sind erfindungsgemäße neue Verbindungen; sie können nach dem erfindungsgemäßen Verfahren (c) hergestellt werden.

Verfahren (d) wird unter Verwendung eines Chlorierungsmittels durchgeführt. Es können die üblichen Mittel für die Umsetzung von Carbonsäuren zu Carbonsäurechloriden eingesetzt werden. Als Beispiele hierfür seien Phosgen, Thionylchlorid, Phosphorylchlorid und Benzotrichlorid genannt. Vorzugsweise wird Thionylchlorid als Chlorierungsmittel verwendet.

Verfahren (d) wird gegebenenfalls in Gegenwart eines Katalysators duchgeführt. Es können die für die Herstellung von Säurechloriden aus Säuren üblichen Katalysatoren, wie z. B. Pyridin oder Dimethylformamid verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Chlorierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Als Ausgangsstoffe für das erfindungsgemäße Verfahren (e) seien genannt:
α-(5-(3-Fluor-5-trifluormethyl-pyridin-2-yl-oxy)-naphthalin-1-yl-oxy)-essigsäure-, -propionsäure- und -buttersäure-chlorid.

Diese Ausgangsverbindungen für Verfahren (e) sind erfindungsgemäße neue Verbindungen; sie können nach dem erfindungsgemäßen Verfahren (d) hergestellt werden.

Die beim erfindungsgemäßen Verfahren (e) weiter als Ausgangsstoffe einzusetzenden Verbindungen sind durch die Formel (VI) allgemein definiert. In Formel (VI) hat Z vorzugsweise bzw. insbesondere diejenige Bedeutung, die bereits oben im Zusammenhang mit der Beschreibung der erfindungsgemäßen

Verbindungen der Formel (I) vorzugsweise bzw. als insbesondere bevorzugt angegeben wurde.

Als Beispiele für die Ausgangsstoffe der Formel (VI) seien genannt:
Methylamin, Ethylamin, Propylamin, Isopropylamin, Anilin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthioethanol, 2-Benzyloxy-ethanol, 3-Benzyloxy-propanol, 2-Benzylthio-ethanol, Hydroxymethanphosphonsäurediethylester und -dimethylester, 1-Hydroxy-ethan-phosphonsäure-dimethylester und -diethylester, 1-Hydroxy-1-phenyl-methanphosphonsäure-dimethylester und -diethylester, Acetonoxim, 3-Hydroxyfuran, Furfurylalkohol, Perhydrofurfurylalkohol, Milchsäure-methylester und -ethylester und Glycolsäuremethylester und -ethylester.

Diese Verbindungen sind bekannte Synthesechemikalien.

Verfahren (e) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verfahren (e) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (e) in einem größeren Bereich variiert werden. Im allgemeinen arbeitet man bei Temperaturen zwischen -20 °C und +100 °C, vorzugsweise bei Temperaturen zwischen 0 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden (vgl. die Herstellungsbeispiele).

Als Ausgangsstoffe für das erfindungsgemäße Verfahren (f) seien genannt:
α-(5-(3-Fluor-5-trifluormethyl-pyridin-2-yl-oxy)-naphthalin-1-yl-oxy)-essigsäure, -propionsäure und -buttersäure, sowie deren Methylester und Ethylester.

Diese Ausgangsstoffe für Verfahren (f) sind erfindungsgemäße neue Verbindungen; sie können nach den erfindungsgemäßen Verfahren (a), (b) oder (c) hergestellt werden.

Die beim erfindungsgemäßen Verfahren (f) weiter als Ausgangsstoffe einzusetzenden Verbindungen sind durch die Formel (VII) allgemein definierte. In Formel (VII) steht vorzugsweise $R^1$ für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl oder $C_1$-$C_4$-Alkoxy-carbonyl-$C_1$-$C_2$-alkyl.

Die Ausgangstoffe der Formel (VII) sind bekannte Synthesechemikalien.

Verfahren (f) wird vorzugsweise unter Verwendung eines Vedünnungsmittels durchgeführt. Als solche kommen insbesondere die Hydroxyverbindungen der Formel (VII) in Betracht, welche bei Verfahren (f) als Reaktionskomponenten eingesetzt werden.

Verfahren (f) wird vorzugsweise in Gegenwart eines Katalysators durchgeführt. Als solche kommen vorzugsweise starke Säuren, wie z.B. Schwefelsäure, Salzsäure, p-Toluolsulfonsäure und Methansulfonsäure in Betracht.

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung von Verfahren (f) setzt man die Ausgangsverbindung der Formel (VII) im allgemeinen im Überschuß ein, so daß sie auch als Verdünnungsmittel dient.

Umsetzung und Aufarbeitung können nach üblichen Methoden durchgeführt werden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen

Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Orysa, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkultern, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in monokotylen und dikotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle seine.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

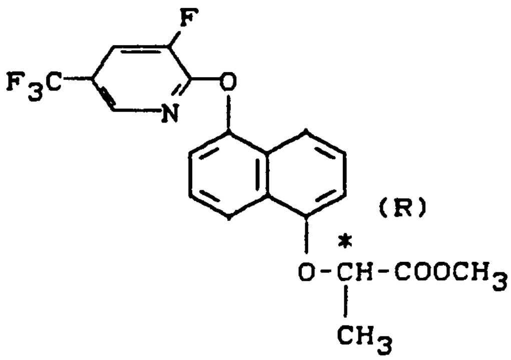

(Verfahren (a))
Eine Mischung aus 4,85 g (15 mMol) 5-(3-Fluor-5-trifluormethyl-pyridin-2-yl-oxy)-1-naphthol, 1,84 g (15 mMol) (S)-α-Chlor-propionsäure-methylester, 3,5 g (25 mMol) Kaliumcarbonat und 120 ml Acetonitril wird 20 Stunden unter Rückfluß erhitzt und dann eingeengt. Der Rückstand wird in Methyl-tert-butylether aufgenommen, mit Wasser gewaschen, mit Natriumsulfat getrocknet, mit Kieselgel verrührt und filtriert. Das Filtrat wird eingeengt, der Rückstand mit Methanol verrührt und das kristallin angefallene Produkt durch Absaugen isoliert.

Man erhält 1,7 g (28% der Theorie) (R)-α-(5-(3-Fluor-5-trifluormethyl-pyridin-2-yl-oxy)-naphthalin-1-yl-oxy)-propionsäure-methylester vom Schmelzpunkt 87 °C.

Beispiel 2

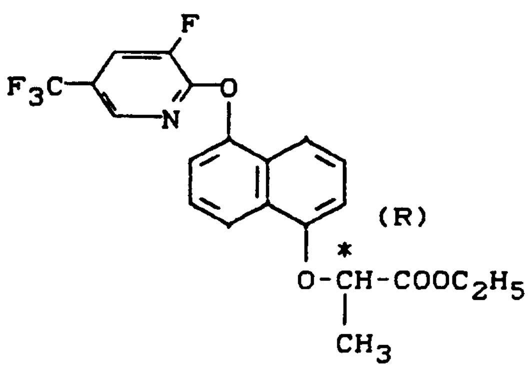

(Verfahren (a))
Eine Mischung aus 1,62 g (5 mMol) 5-(3-Fluor-5-trifluormethyl-pyridin-2-yl-oxy)-1-naphthol, 1,36 g (5 mMol) (S)-α-(4-Methyl-phenylsulfonyloxy)-propionsäure-ethylester, 1,0 g (7,5 mMol) Kaliumcarbonat und 60 ml Acetonitril wird 20 Stunden unter Rückfluß erhitzt und dann eingeengt. Der Rückstand wird in Methyl-tert-butylether aufgenommen, mit Wasser gewaschen, mit Natriumsulfat getrocknet, mit Kieselgel verrührt und filtriert. Das Filtrat wird eingeengt, der Rückstand mit Methanol verrührt und das kristallin angefallene Produkt durch Absaugen isoliert.

Man erhält 1,8 g (85% der Theorie) (R)-α-(5-(3-Fluor-5-trifluormethyl-pyridin-2-yl-oxy)-naphthalin-1-yl-oxy)-propionsäure-ethylester vom Schmelzpunkt 128 °C.

Beispiel 3

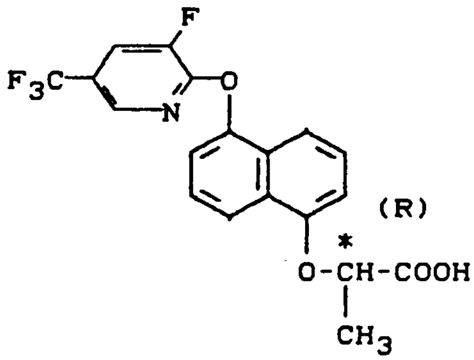

(R)

(Verfahren (c))

Eine Mischung aus 0,72 g (1,75 mMol) (R)-α-(5-(3-Fluor-5-trifluormethyl-pyridin-2-yl-oxy)-naphthalin-1-yl-oxy)-propionsäure-methylester, 0,12 g (2,6 mMol) Natriumhydroxid und 60 ml Wasser wird 20 Stunden unter Rückfluß erhitzt und nach Abkühlen mit konzentrierter Salzsäure angesäuert. Das kristallin angefallene Produkt wird durch Absaugen isoliert.

Man erhält 0,5 g (72% der Theorie) (R)-α-(5-(3-Fluor-5-trifluormethyl-pyridin-2-yl-oxy)-naphthalin-1-yl-oxy)-propionsäure vom Schmelzpunkt 234 ° C.

Analog zu den Herstellungsbeispielen 1 bis 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

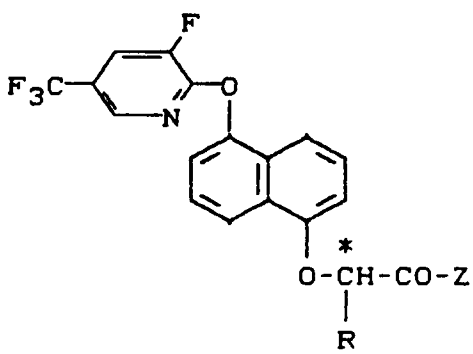

(I)

Tabelle 1: Beispiele für die Verbindungen der Formel (I)

| Bsp.-Nr. | R | Z | Konfiguration an * | | Physikal. Daten | |
|---|---|---|---|---|---|---|
| 4 | $CH_3$ | $-OC_3H_7n$ | (R) | t(3H) | δ = 0,87 | ($CDCl_3$) |
| 5 | $CH_3$ | $-OCH_2$ 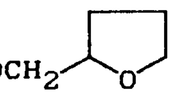 | (R/S) | q (1H) | δ = 5,01 | ($CDCl_3$) |
| 6 | $CH_3$ | 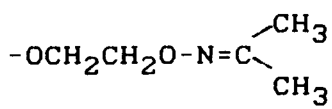 | (R/S) | q (1H) | δ = 4,97 | ($CDCl_3$) |
| 7 | $CH_3$ | $-(OCH_2CH_2)_2OCH_3$ | (R/S) | s (3H) | δ = 3,35 | ($CDCl_3$) |
| 8 | $C_2H_5$ | $OCH_3$ | (R/S) | m (1H) | δ = 2,16 | ($CDCl_3$) |
| 9 | $CH_3$ | $OCH_2CH_2OC_2H_5$ | | | | |
| 10 | $CH_3$ | $OCH_2CH_2OCH_2$ 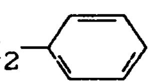 | | | | |

<u>Tabelle 1:</u> (Fortsetzung)

| Bsp.-Nr. | R | Z | Konfiguration an * | Physikal. Daten | | |
|---|---|---|---|---|---|---|
| 11 | $CH_3$ | $-OCH_2CH_2Si(CH_3)_3$ | (R/S) | $\delta$ = 4,90 | (q, 1H) | ($CDCl_3$) |
| 12 | $CH_3$ | $-OCH(CH_3)_2$ | (R) | $\delta$ = 1,75 | (d, 3H) | ($CDCl_3$) |
| 13 | $CH_3$ | $-OCH_2CH_2SCH_3$ | (R/S) | $\delta$ = 2,67 | (t, 2H) | ($CDCl_3$) |
| 14 | $CH_3$ | $-OCH_2C_6H_5$ | (R/S) | $\delta$ = 5,19 | (s, 2H) | ($CDCl_3$) |
| 15 | $CH_3$ | $-\underset{\underset{CH_3}{\vert}}{N}-OCH_3$ | (R/S) | $\delta$ = 6,79 | (d, 1H) | ($CDCl_3$) |
| 16 | $CH_3$ | $-OCH_2CH(CH_3)_2$ | (R) | $\delta$ = 1,77 | (d, 3H) | ($CDCl_3$) |

Ausgangsstoffe der Formel (II)

Beispiel (II-1)

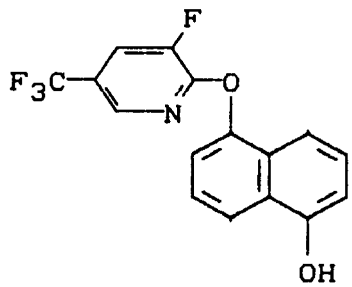

7,6 g (136 mMol) Kaliumhydroxid-Pulver werden zu einer Lösung von 35,8 g (224 mMol) 1,5-Dihydroxynaphthalin in 320 ml Dimethylsulfoxid gegeben und das Gemisch wird eine Stunde bei 20 °C gerührt. Dann werden 14,6 g (80 mMol) 2,3-Difluor-5-trifluormethyl-pyridin dazu gegeben und das Reaktionsgemisch wird 20 Stunden bei 80 °C gerührt. Anschließend wird im Dampfstrahlvakuum eingeengt, der Rückstand mit Wasser aufgenommen, mit Methyl-tert-butylether extrahiert, die Extraktionslösung mit Natriumsulfat getrocknet und filtriert. Der Rückstand wird in Toluol aufgenommen, heiß über Kieselgel filtriert, eingeengt, mit Petrolether verrührt und abgesaugt.

Man erhält 9,6 g (37% der Theorie) 5-(3-Fluor-5-trifluormethyl-pyridin-2-yl-oxy)-1-naphthol vom Schmelzpunkt 161 °C.

Anwendungsbeispiele;

In den folgenden Anwendungsbeispielen werden die nachstehend aufgeführten Verbindungen als Vergleichssubstanzen herangezogen.

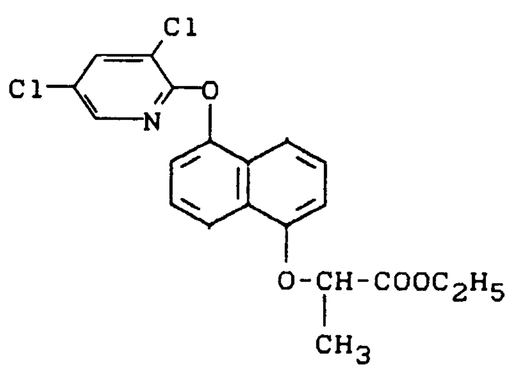

(A)

α-(5-(3,5-Dichlor-pyridin-2-yl-oxy)-naphthalin-1-yl-oxy)-propionsäure-ethylester

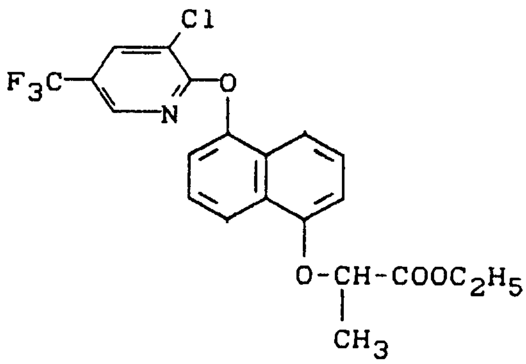

(B)

α-(5-(3-Chlor-5-trifluormethyl-pyridin-2-yl-oxy)-naphthalin-2-yl-oxy)-propionsäure-ethylester (beide bekannt aus JP-A 54032477).

Beispiel A

Post-emergence-Test

Lösungsmittel: 5 Gewichtsteile Aceton
Emulgator; 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik bei guter Nutzpflanzenselektivität zeigen in diesem Test z.B. die Verbindungen gemäß folgender Herstellungsbeispiele: 1 und 3, welche bei einer Aufwandmenge von 500 g/ha eine 100 %ige Wirkung gegen Echinochloa, Setaria und Sorghum zeigen.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber dem Stand der Technik bei guter Nutzpflanzenselektivität zeigen in diesem Test z.B. die Verbindungen gemäß folgender Herstellungsbeispiele: 1 und 3.

Beispiel C Test an verpflanztem Wasserreis

Lösungsmittel: 5 Gewichtsteile Aceton
Emulgator: 1 Gewichtsteil Benzyloxypolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat auf die gewünschte Konzentration.

Pflanzgefäße (Oberfläche 1/5000 Ar) werden mit Boden aus einem Reisfeld gefüllt. Zwei Reispflanzen (Sorte; Kinmaze) werden im 2-3 Blattstadium (ca. 10 cm hoch) in die Gefäße verpflanzt. Samen von Echinochloa crus galli und/oder Monochoria vaginalis und/oder kleine Rhizomabschnitte von Eleocharis acicularis L. werden in die feucht gehaltene Erde ausgesät. 2 Tage nach dem Verpflanzen des Reises wird der Boden bis zu einer Wassertiefe von 3 cm überstaut. Die Wirkstoffzubereitung wird auf die Wasseroberfläche ausgebracht. Die Wirkstoffkonzentration in der Zubereitung spielt keine Rolle, entscheidend ist nur die Aufwandmenge des Wirkstoffs pro Flächeneinheit`

Nach der Anwendung des Wirkstoffs wird für 2 Tage durch die Pflanzgefäße ein vertikal absteigender Wasserstrom mit einer Geschwindigkeit von 2-3 cm pro Tag eingestellt. Danach werden die Testansätze unter Überflutungsbedingungen gehalten, wobei die Wassertiefe 3 cm beträgt.

Nach 4 Wochen wird der Schädigungsgrad der Pflanzen bonitiert in % Schädigung (bzw. Unkrautwirkung) im Vergleich zu einer unbehandelten Kontrolle.

Es bedeuten:
0% = keine Wirkung (wie unbehandelte Kontrolle)
100% = totale Vernichtung

In diesem Test zeigen z.B. die Verbindungen gemäß den Herstellungsbeispielen 1 und 2 bei guter Verträglichkeit gegenüber Reis bei einer Aufwandmenge von 1000 g/h eine 100 %ige Wirkung gegen Hühnerhirse (Echinochloa crus galli).

**Patentansprüche**

**1.** Pyridyloxy-naphthaline der Formel (I)

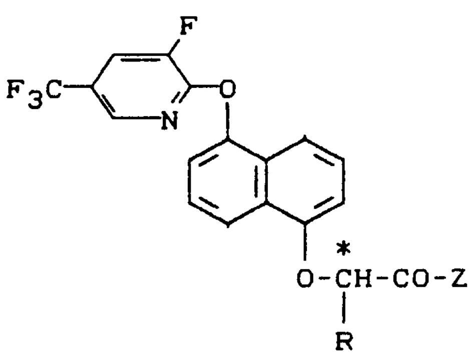

(I)

in welcher

R für Wasserstoff oder $C_1$-$C_4$-Alkyl steht und

Z für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, N-($C_1$-$C_6$-Alkoxy)-N-($C_1$-$C_4$-alkyl)-amino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxycarbonyl-$C_1$-$C_2$-alkylthio oder für die Gruppierung -O-$R^1$ steht, worin

$R^1$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Trimethylsilylethyl, Phenylthio-$C_1$-$C_3$-alkyl, Benzyloxy$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino, $C_2$-$C_4$-

Alkylidenaminooxy-$C_1$-$C_4$-alkyl, oder für ein Ammonium-, ein $C_1$-$C_4$-Alkylammonium-, ein Natrium-, Kalium- oder Calcium-äquivalent steht, oder für
die Gruppierung

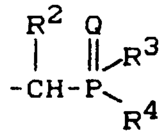

steht, worin

R² für Wasserstoff, $C_1$-$C_4$-Alkyl, Phenyl, Furyl, Thienyl oder Pyridyl steht,
R³ für $C_1$-$C_4$-Alkyl oder $C_1$-$C_4$-Alkoxy steht,
R⁴ für $C_1$-$C_4$-Alkoxy steht und
Q für Sauerstoff oder Schwefel steht,
oder
R¹ für die Gruppierung $(-CH_2-)_n-R^5$ steht,
worin
n für die Zahlen 0, 1 oder 2 steht und
R⁵ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Isoxazolidinyl, Pyridinyl oder Pyrimidinyl steht,

**2.** Verbindungen der Formel (I) gemäß Anspruch 1, in welcher

R für Wasserstoff, Methyl oder Ethyl steht und
Z für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, N-($C_1$-$C_4$-Alkoxy)-N-($C_1$-$C_3$-alkyl)-amino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^1$ steht, worin
R¹ für $C_1$-$C_4$-Alkyl, $C_1$-$C_2$-Alkoxy-$C_1$-$C_2$-alkyl, $C_1$-$C_2$-Alkoxy-$C_1$-$C_2$-alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl, Trimethylsilylethyl, $C_2$-$C_4$-Alkylidenaminooxy-$C_2$-$C_3$-alkyl oder für ein Ammonium-, $C_1$-$C_3$-Alkylammonium-, Natrium- oder Kaliumäquivalent steht,
oder für
die Gruppierung

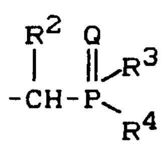

steht,
worin

R² für Wasserstoff, Methyl, Phenyl, Furyl Thienyl oder Pyridyl steht,
R³ für Methoxy oder Ethoxy steht,
R⁴ für Methoxy oder Ethoxy steht und
Q für Sauerstoff oder Schwefel steht,
oder
R¹ für die Gruppierung $-(CH_2)_n-R^5$ steht, worin
n für die Zahlen 0, 1 oder 2 steht und
R⁵ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl, Isoxazolidinyl und Dioxolanyl steht.

3. Verfahren zur Herstellung von Pyridyloxy-naphthalinen der Formel (I), dadurch gekennzeichnet, daß man

(a) 5-(3-Fluor-5-trifluormethyl-pyridin-2-yl-oxy)-1-naphthol der Formel (II)

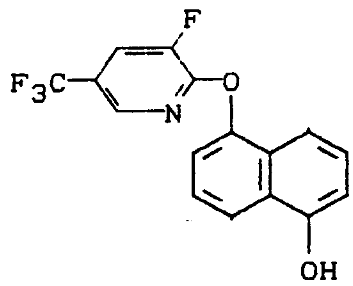

(II)

mit Carbonsäurederivaten der allgemeinen Formel (III)

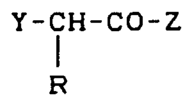

(III)

in welcher

R und Z die oben angegebene Bedeutung haben und

Y für eine nucleophile Abgangsgruppe steht,

gegebenenfalls in Gegenwart eines Säureakzeptors und gegebenenfalls in Gegenwart eines Verdünnungsmittels umsetzt, oder

(b) Halogen-pyridin-Derivate der allgemeinen Formel (IV)

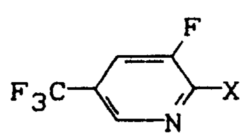

(IV)

in welcher

X für Halogen steht,

mit Hydroxynaphthyloxycarbonsäure-Derivaten der allgemeinen Formel (V)

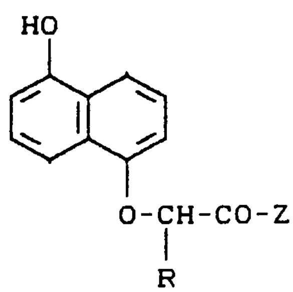

(V)

in welcher

R und Z die oben angegebene Bedeutung haben,

gegebenenfalls in Gegenwart eines Säureakzeptors und gegebenenfalls in Gegenwart eines Verdünnungsmittels umsetzt, oder

(c) für den Fall, daß Z für Hydroxy steht, die Verbindungen der Formel (I), in welcher Z für Methoxy oder Ethoxy steht, mit einem Alkalihydroxid in Gegenwart von Wasser und gegebenenfalls in Gegenwart eines organischen Lösungsmittels umsetzt und dann - gegebenenfalls nach Einengen -

mit einer Mineralsäure ansäuert, oder

(d) für den Fall, daß Z für Chlor steht, die Verbindungen der Formel (I), in welcher Z für Hydroxy steht, mit einem Chlorierungsmittel gegebenenfalls in Gegenwart eines Katalysators und gegebenenfalls in Gegenwart eines Verdünnungsmittels umsetzt, oder

(e) für den Fall, daß Z mit Ausnahme von Chlor die oben angegebene Bedeutung hat, die Verbindungen der Formel (I), in welcher Z für Chlor steht, mit Verbindungen der allgemeinen Formel (VI)

H-Z (VI)

in welcher

Z mit Ausnahme von Chlor die oben angegebene Bedeutung hat,

gegebenenfalls in Gegenwart eines Säureakzeptors und gegebenenfalls in Gegenwart eines Verdünnungsmittels umsetzt, oder

(f) für den Fall, daß Z für die Gruppierung -O-$R^1$ steht, worin $R^1$ mit Ausnahme von Ammonium, Alkylammonium, Alkalimetall und Erdalkalimetall die oben angegebene Bedeutung hat, Verbindungen der Formel (I), in welcher Z für Hydroxy, Methoxy oder Ethoxy steht,
mit Hydroxyverbindungen der allgemeinen Formel (VII)

HO-$R^1$ (VII)

in welcher

$R^1$ mit Ausnahme von Ammonium, Alkylammonium, Alkalimetall und Erdalkalimetall die oben angegebene Bedeutung hat,

gegebenenfalls in Gegenwart eines Katalysators und gegebenenfalls in Gegenwart eines Verdünnungsmittels umsetzt.

**4.** Herbizide Mittel, gekennzeichnet durch einen Gehalt an mindestens einem Pyridyloxy-naphthalin der Formel (I) gemäß den Ansprüchen 1 bis 3.

**5.** Verfahren zur Bekämpfung von Unkräutern, dadurch gekennzeichnet, daß man Pyridyloxy-Naphthaline der Formel (I) gemäß den Ansprüchen 1 bis 3 auf die Unkräuter und/oder ihren Lebensraum einwirken läßt.

**6.** 5-(3-Fluor-5-trifluormethyl-pyridin-2-yl-oxy)-1-naphthol der Formel (II)

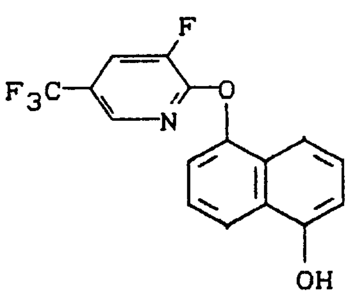

(II)

**7.** Verwendung von Pyridyloxy-naphthalinen der Formel (I) gemäß den Ansprüchen 1 bis 3 zur Bekämpfung von Unkräutern.

**8.** Verfahren zur Herstellung von herbiziden Mitteln, dadurch gekennzeichnet, daß man Pyridyloxy-naphthaline Derivate der Formel (I) gemäß den Ansprüchen 1 bis 3 mit Streckmitteln und/oder oberflächenaktiven Substanzen vermischt.

Europäisches Patentamt

# EUROPÄISCHER RECHERCHENBERICHT

Nummer der Anmeldung
EP 93 10 0337

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X,D | CHEMICAL ABSTRACTS, vol. 91, no. 11, 10. September 1979, Columbus, Ohio, US; abstract no. 91510j, R. NISHIYAMA ET AL. 'Pyridyloxynaphthoxypropionic acid derivatives' Seite 767 ; * Zusammenfassung * & JP-A-54 032 477 (ISHIHARA SANGYO KAISHA, LTD.) | 1-8 | C07D213/64<br>C07D405/12<br>C07F7/10<br>A01N43/40<br>A01N43/08<br>//(C07D405/12, 307:00, 213:00) |
| A | EP-A-0 347 679 (BAYER AG) * Seite 6, Zeile 50 - Seite 7, Zeile 13; Ansprüche * | 1-8 | |
| A | EP-A-0 315 008 (BAYER AG) * Ansprüche; Tabelle 1 * | 1-8 | |
| A | EP-A-0 288 275 (TOSOH CORPORATION) * Ansprüche 1,7-9; Beispiel 14; Tabellen 2,3 * | 1-8 | RECHERCHIERTE SACHGEBIETE (Int. Cl.5 ) |
| A | EP-A-0 179 015 (SCHERING AKTIENGESELLSCHAFT) * Ansprüche; Beispiel 42 * | 1-8 | C07D |

Der vorliegende Recherchenbericht wurde für alle Patentansprüche erstellt

| Recherchenort | Abschlußdatum der Recherche | Prüfer |
|---|---|---|
| DEN HAAG | 17 MAERZ 1993 | P. BOSMA |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)